(19) Europäisches Patentamt
European Patent Office
Office européen des brevets

(11) Veröffentlichungsnummer: **0 019 829**
**B1**

(12) **EUROPÄISCHE PATENTSCHRIFT**

(45) Veröffentlichungstag der Patentschrift: **16.11.83**

(21) Anmeldenummer: **80102764.0**

(22) Anmeldetag: **20.05.80**

(51) Int. Cl.³: **C 07 C 103/52,**
**A 61 K 37/02,**
**C 12 P 21/02, C 12 P 21/06**

# (54) Pharmakologisch aktive Peptide und deren Zusammensetzungen.

(30) Priorität: **25.05.79 DE 2921216**

(43) Veröffentlichungstag der Anmeldung:
**10.12.80 Patentblatt 80/25**

(45) Bekanntmachung des Hinweises auf die Patenterteilung:
**16.11.83 Patentblatt 83/46**

(84) Benannte Vertragsstaaten:
**AT BE CH FR GB IT LI LU NL SE**

(56) Entgegenhaltungen:
**DE - A - 2 328 093**
**FR - A - 2 298 334**
**JP - B - 73 043 906**

**UNLISTED DRUGS, Februar 1980 (Edit. Spec. Library Association) Naunym Schmied Arch Pharmacol 308 (Suppl): R 39 (155 Abs), 1979 Casomorphins**

(73) Patentinhaber: **Brantl, Victor, Dipl.-Chem.**
**Frauenplatz 10**
**D-8000 München 2 (DE)**

(73) Patentinhaber: **Teschemacher, Hansjörg, Prof. Dr.**
**Pharmakologisches Institut der Universität Giessen Frankfurter Strasse 107**
**D-6300 Giessen 1 (DE)**

(73) Patentinhaber: **Henschen, Agnes, Dr.**
**Max Planck Institut für Biochemie**
**D-8033 Martinsried (DE)**

(73) Patentinhaber: **Lottspeich, Friedrich, Dr.**
**Max Planck Institut für Biochemie**
**D-8033 Martinsried (DE)**

(72) Erfinder: **Brantl, Victor, Dipl.-Chem.**
**Frauenplatz 10**
**D-8000 München 2 (DE)**
Erfinder: **Teschemacher, Hansjörg, Prof. Dr.**
**Pharmakologisches Institut der Universität Giessen Frankfurter Strasse 107**
**D-6300 Giessen 1 (DE)**
Erfinder: **Henschen, Agnes, Dr.**
**Max Planck Institut für Biochemie**
**D-8033 Martinsried (DE)**
Erfinder: **Lottspeich, Friedrich, Dr.**
**Max Planck Institut für Biochemie**
**D-8033 Martinsried (DE)**

(74) Vertreter: **Schacht, Wilhelm, Dr. jur.**
**Am Eselsweg 47**
**D-6500 Mainz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 019 829 B1

## Pharmakologisch aktive Peptide und deren Zusammensetzungen

Die Erfindung betrifft neue pharmakologisch aktive Peptide, insbesondere opiatartig wirkend, Verfahren zu deren Aktivierung und/oder Isolierung, Verfahren zu deren synthetischer Herstellung, sowie nach diesen Verfahren hergestellte pharmakologisch aktive, insbesondere opiatartig wirkende Peptide.

Es sind pharmakologisch aktive Peptide bekannt, welche in besonderen biologischen Testsystemen eine spezifische opiatartige Wirkung entfalten. Es sind dies insbesondere die von Hughes et al. (Nature 258, 577, 1975) aus dem Gehirn isolierten Pentapeptide: das Methionin- und das Leucinenkephalin (Tyr-Gly-Gly-Phe-Met und Tyr-Gly-Gly-Phe-Leu) sowie deren längerkettigen Analoga. Diese bekannten Peptide und deren synthetischen Derivate weisen den Nachteil auf, daß diese nach in vitro Behandlung mit Proteasen (z.B. Pronase E, Merck) nach kurzer Inkubationszeit ihre pharmakologische Wirkung, in diesem Fall eine opiatartige, verlieren. Bei in vivo Verabreichung werden diese Peptide durch körpereigene Proteasen leicht angegriffen und zerstört.

Zu den oben genannten Enkephalinen gehören auch die in der französischen Patentanmeldung FRA 23 59 58 17 (1977) beschriebenen opiatartig wirkenden Peptide.

Die in der vorliegenden Erfindung beschriebenen Peptide und deren Analoga, die zur Klasse der $\beta$-Casomorphine gehören, weisen eine grundsätzlich andere Basisstruktur, d.h. Aminosäuresequenz auf. Aufgrund dieser neuen Struktur weisen die $\beta$-Casomorphine und deren Analoga im Vergleich zu den Enkephalinen unterschiedliche biochemische und pharmakologische Eigenschaften auf (Brantl et al., Henschen et al., Hoppe Seyler's Z. Physiol. Chem. 360, 1211—1216 und 1217—1224 (1979); Brantl et al., Life Sci. 28, 1903—1909, (1981), Chang et al., Science 212, 75—77 (1981)).

Die deutsche Patentanmeldung DE—A—23 28 093 (1973) betrifft Peptidinhibitoren mit 8—10 Aminosäuren. Sie enthält keine Anleitung zur Schaffung opiataktiver Peptide und auch keine Anleitung zur Verknüpfung von Aminosäuren (Aminosäuresequenz), die zu einem opiataktiven Peptid führen, das besonders stabil gegenüber proteolytischen Enzymen ist. Opiataktive Peptide wurden erst 1975 von Hughes et al. (Nature 258, 577, 1975) entdeckt.

Schließlich sei noch die Druckschrift J.O.C. 28, Nr. 4 (1962), Seite 1028 erwähnt. In dieser Druckschrift wird zwar ein bestimmtes Tripeptid (Tyr-Pro-Phe) beschrieben, aber ohne Angaben von Daten über die opiatartige Wirksamkeit dieses Peptids.

In den Schutzumfang der vorliegenden Erfindung fallen lediglich Tripeptid-Ester und -Amide und betreffen so nicht das in J.O.C., 28, (1962) genannte Tripeptid.

Aufgabe der vorliegenden Erfindung ist es, neue pharmakologisch aktive Peptide, insbesondere opiatartig wirkende, zu schaffen, welche eine neue, besondere Aminosäuresequenz aufweisen und aufgrund dieser Sequenz über-rasschenderweise eine erhöhte Stabilität gegenüber peptidspaltenden Enzymen (Proteasen, z.B. Pronase E, Merck) aufweisen. Aufgabe der Erfindung ist es weiterhin, den Fachmann durch die Offenbarung der Aminosäuresequenz der pharmakologisch aktiven Peptide in die Lage zu versetzen, mit üblichen Methoden das Peptid in beliebiger Menge herzustellen.

Diese Aufgabe ist gemäß der Erfindung dadurch gelöst, daß die opiatartig wirkenden Peptide folgende Formel aufweisen:

V-Pro-A-X

in dieser steht

Pro für Prolin

A für jede beliebige Aminosäure, auch Prolin und Tyrosin

X für

a) C-terminale Ester oder Amide des Tripeptids V-Pro-A

b)

Pro-Y
Pro-B-Y
Pro-B-Pro-Y
Pro-B-Pro-C-Y
Pro-B-Pro-C-Pro-Y

wobei

B und C jede beliebige Aminosäure sein kann, auch Prolin und Tyrosin. Y steht für die freie Säurefunktion der jeweiligen C-terminalen Aminosäure oder deren Ester oder deren Amide.

V für die N-terminale Aminosäure Tyrosin oder für das N-terminal endständige Tyrosin der allgemeinen Formel

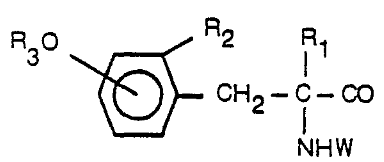

wobei im einzelnen bedeuten:

$R_1$ = Wasserstoff oder eine Alkylgruppe mit 1 bis 4 C-Atomen,
$R_2$ = Wasserstoff oder zusammen mit $R_1$ für eine Aethylenbrücke,
$R_3$ = Wasserstoff, eine Alkylgruppe mit 1 bis 4 C-Atomen oder eine $R_4CO$-Gruppe,
$R_4$ = einen gesättigten oder ungesättigten, geradkettigen oder verzweigten Alkylrest mit 1 bis 17 C-Atomen, einen Phenylrest oder einen Phenylalkylrest mit 7 bis 12 C-Atomen, wobei die Phenylrest durch 1 oder 2 Substituenten aus der Reihe Halogen, Alkyl mit 1 bis 4 C-Atomen oder Alkoxy mit 1 bis 4 C-Atomen substituiert sein können, wobei die $R_3O$-Gruppe sich in meta- oder para-Stellung zum

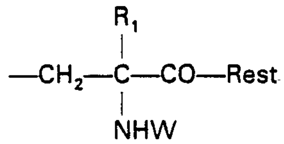

befindet,

W = Wasserstoff, Alkyl mit 1 bis 5 C-Atomen, Alkenyl mit 3 bis 5 C-Atomen, Cyclopropylmethyl, Cyclobutylmethyl, $R_4CO$-, H-Lys-, H-Phe- oder H-Tyr.

Nach einer Weiterbildung der Erfindung sind die Buchstaben A, B, C, der bisher aufgeführten Peptide:

A Phenylalanin
B Glycin
C Isoleucin

Nach einer anderen Weiterbildung ist

a) das Phenylalanin der allgemeinen Formel:

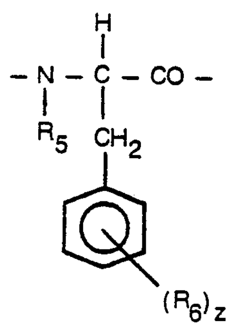

durch

$R_5$ für Wasserstoff oder Alkyl mit 1 bis 4 C-Atomen,
$R_6$ für Wasserstoff, Fluor, Chlor, Brom, Nitro, Alkyl mit 1 bis 4 C-Atomen oder Alkoxy mit 1 bis 4 C-Atomen,
z für 1 oder 2 ersetzt.

b) das Prolin der allgemeinen Formel:

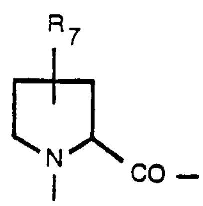

so modifiziert ist, daß:

$R_7$ für Wasserstoff, eine Hydroxygruppe, eine Alkyl- oder Alkoxygruppe mit 1 bis 4 C-Atomen steht.

— am Stickstoff eine Alkyl- oder Alkoxygruppe mit 1 bis 4 C-Atomen gebunden ist.

— eine oder mehrere Ketogruppen in den Ring eingeführt sind.

Nach einer Weiterbildung sind die optisch aktiven Aminosäuren und/oder Aminosäurereste der Peptide durch solche ersetzt, welche stereochemisch die D-, L-, oder D/L Konfiguration aufweisen.

Nach einer bevorzugten Weiterbildung liegen alle Aminosäuren des Peptids in der L-Form vor.

Die erfindungsgemäßen Peptide weisen ebenso vorteilhafte wie erstaunliche Proteasenstabilität auf. Diese Proteasenstabilität ist vermutlich ganz oder teilweise auf den Einbau der Aminosäure Prolin

in die Peptidkette zurückzuführen; somit wird der Angriff von Proteasen behindert. Diese Stabilität gegenüber den Proteasen kann insbesondere für die pharmakologische Applikation von Vorteil sein, da man eine längere Wirkung mit kleineren Dosen erzielen kann.

Ein weiterer Vorteil der erfindungsgemäßen pharmakologisch aktiven Peptide ist die Steuerbarkeit der pharmakologischen Wirksamkeit, hier eine opiatartige, über die Kettenlänge; so ist das erfindungsgemäße Peptid mit 5 Aminosäuren wesentlich wirksamer als das mit 7 Aminosäuren, bezogen auf molare Konzentrationen der Stoffe im Vergleich zu Normorphin (siehe Ausführungsbeispiel).

Nach einer Weiterbildung der Erfindung werden die erfindungsgemäßen Peptide aus natürlichen Quellen unter Anwendung allgemein üblicher Methoden erzeugt und anschließend isoliert. Die pharmakologisch aktiven Peptide können aus in Tier- und Pflanzenreich vorkommenden Proteinen durch Enzyme freigesetzt und so aktiviert werden.

Nach einer Weiterbildung werden die pharmakologisch aktiven Peptide aus $\alpha$- und/oder $\beta$- und/oder $\varkappa$-Casein durch Enzyme freigesetzt und aktiviert. Diese ist besonders vorteilhaft, da das Casein weder selbst, noch dessen Chloroform/Methanol (65/35, Vol/Vol) Extrakte eine pharmakologische Wirkung, hier eine opiatartige, zeigen.

Nach einer besonders bevorzugten Weiterbildung sind die Enzyme pankreatischen Ursprungs.

Nach einer weiteren vorteilhaften Ausgestaltung der Erfindung sind die Enzyme Trypsin, Chymotrypsin, Pronase E, Thermolysin einzeln und/oder deren Gemische.

Die Freisetzung der pharmakologisch aktiven Peptide durch die o.g. Enzyme bedingt zugleich auch noch die Eigenschaft der erfindungsgemäßen Peptide, nämlich von diesen Enzymen nicht oder nur wenig angegriffen zu werden.

Nach einer Weiterbildung der Erfindung werden die pharmakologisch aktiven Peptide aus einem enzymatisch verdauten Casein mittels eines oder mehrerer der folgenden Schritte gewonnen:

a) einer Extraktion mit Chloroform/Methanol (65/35; Vol/Vol)

b) Adsorption des eingedampften Chloroform/Methanolextraktes aus wässriger Lösung an Aktivkohle. Desorption mit reinem Chloroform.

c) Adsorption des eingedampften Chloroformextraktes aus wässriger Lösung an Amberlite[R] (XAD—2, 300—1000 mesh). Desorption mittels Methanol.

d) weitere Reinigung des Methanolextraktes mittels einer Hochdruckflüssigkeitschromatochraphieanlage (HPLC) über eine sog. "reversed phase" Säule (Füllmaterial: Bondapak $C_{18}$, Waters) und über eine Silicagelsäule (Füllmaterial: $\mu$-Porasil, Waters) in lipophilen und/oder hydrophilen Lösungsmitteln.

e) Chromatographie auf einer Biogel P2 Säule.

Das verwendete enzymatische Caseinhydrolysat kann Serva C/TLG 16300 und/oder C/PLB 16322 sein. Hier sei noch angemerkt, daß andere Caseinhydrolysate wie z.B. Serva C/HSF 16320 und C/HSH 16322 keine extrahierbaren pharmakologisch wirksamen Peptide enthalten.

Die opiatartige Wirkung kann durch Inkubation mit Carboxypeptidase (siehe Ausführungsbeispiel) verstärkt werden.

Nach einer Weiterbildung der Erfindung werden die erfindungsgemäßen pharmakologisch aktiven Peptide mit einem in der Peptidchemie und/oder in der Natur üblichen Verfahren synthetisiert.

Unter üblichen Verfahren werden das Einführen sowie das Abspalten von Schutzgruppen (z.B. der Schutz der Aminogruppe durch Carbobenzoxychlorid; der Schutz von Säuregruppen als Ester) während der Synthese verstanden. Üblich sind ebenfalls Synthesen an Trägermaterialien wie z.B. Polystyrol.

Nach einer Weiterbildung der Erfindung werden die pharmakologisch aktiven Peptide durch Knüpfen der Peptidbindung zwischen den einzelnen Aminosäuren und/oder den kleineren Peptidfragmenten mit den üblichen Methoden erzeugt.

Die erfindungsgemäßen pharmakologisch wirksamen Peptide, nach einem der Ansprüche 1—14, und/oder deren Derivate und/oder deren Salze können in Arznei- und Tierarzneimitteln enthalten sein.

Diese können insbesondere als Antitussiva, Antidiarrhoica, Analgetica, Antipsychotica, Tranquilizer Verwendung finden.

Nach einer Weiterbildung der Erfindung werden die in den Ansprüchen 1—14 charakterisierten Peptide als Diagnostica verwendet.

Dies kann z.B. in der Bestimmung der opiatartig wirkenden Stoffe in der Milch und/oder deren Produkten als Indikator des Mikroorganismenbefalls dienen.

Nach einer Weiterbildung der Erfindung werden die erfindungsgemäßen Peptide nach Ansprüchen 1—14, nach Kopplung an Thyreoglobulin oder andere makromoleckulare Eiweißkörper, als Antigene zur Bildung von Antikörpern im Säugetierorganismus gegen die in den Ansprüchen 1—14 beschriebenen Peptide verwendet, und diese Antikörper wiederum zur Bestimmung der Konzentration derselben in Körpergeweben und -Flüssigkeiten.

Dabei wird das Peptid in üblicher Weise mit Carbodiimid an das Makromolekül gekoppelt und intracutan in die Rücken- und Bauchhaut eines Kanischens injiziert.

Nach einer Weiterbildung der Erfindung können die Derivate der erfindungsgemäßen Peptide nach Ansprüchen 1—7 Opiatantagonisten verwendet werden.

## Ausführungsbeispiele

Die vorliegende Erfindung wird anhand zweier Ausführungsbeispiele beschrieben. Das erste beschreibt die Isolierung eines Heptapeptids mit opiatartiger Wirkung aus enzymatisch gespaltenem Casein. Das zweite beschreibt die Totalsynthese eines opiatartig wirkenden Pentapeptids.

### Beispiel I

Isolierung eines Heptapeptids mit opiatartiger Wirkung aus enzymatisch gespaltenem Casein.

Alle Überstände und Filtrate aus den im folgenden beschriebenen Extraktions- und Adsorptionsprozeduren, sowie alle Fraktionen aus der Hochdruckflüssigkeitschromatographie und aus der Gel-Filtration wurden mittels des später beschriebenen Verfahrens auf ihren Gehalt an opiataktiven Stoffen getestet.

#### Schritt 1: Chloroform/Methanol-Extraction

442 g eines Rinder-Casein-Peptons (Casein-Spaltprodukte nach Enzymbehandlung, Serva, C/TLG 16300) wurden mit ca. 3 Liter Chloroform/Methanol (65/35; v/v) versetzt und diese Suspension über Nacht mittels eines Magnetrührers gerührt. (Dieser und die folgenden Reinigungsschritte wurden bei Zimmertemperatur durchgeführt). Die Suspension wurde danach durch Papierfaltendfilter (Schleicher — Schüll) filtriert und das Filtrat mittels eines Rotationsverdampfers zur Trockene gebracht.

#### Schritt 2: Aktivkohle-Adsorption

Der Verdampfungsrückstand aus Schritt 1 wurde mit Aktivkohle (Nr. 2186, Merck) vermischt (Gewichtsverhältnis: 1:1) und dieses Gemisch in Wasser (1/6; Gewicht/Volumen) suspendiert. Nach 10-minütigem Rühren wurde die Suspension zentrifugiert und der Überstand verworfen. Das Sediment aus dieser Zentrifugation wurde in Wasser resuspendiert; diese Suspension wiederum zentrifugiert und der Überstand wiederum verworfen. Dieser Schritt, inklusive Resuspension, Zentrifugation und Verwerfung des Überstandes wurde hierauf noch zweimal wiederholt — jedoch unter Benutzung von Methanol. Die Desorption des opiataktiven Peptids von der Aktivkohle erfolgte nun durch zweimalige Wiederholung der geschilderten Prozedur unter Benutzung von Chloroform. Der Chloroform-Überstand wurde durch Papierfilter filtriert und das Filtrat am Rotationsverdampfer eingedampft. Der Rückstand wurde in einem kleinen Volumen Chloroform/Methanol (1/1; v/v) aufgenommen und durch Mikrofilter (Nr. FHLPO—1300 Millipore) gefiltert, um Aktivkohlerückstände zu entfernen; das Filtrat wurde eingedampft.

#### Schritt 3: Amberlite$^R$ XAD-2-Adsorption

Der Rückstand aus der Verdampfung nach Schritt 2 wurde in Wasser aufgenommen (1/10; Gewicht/Volumen). Zu dieser Lösung wurde Amberlite$^R$ XAD-2-Harz (300—1000 mesh, Serva) im Verhältnis (10/5; Volumen/Gewicht) zugesetzt. Nach 10-minütigem Rühren wurde die Suspension durch Papierfilter filtriert. Der Rückstand, der das Amberlite$^R$-Harz enthielt, an welches das opiataktive Peptid adsorbiert war, wurde mit einem großen Volumen an Wasser (1/25; Gewicht/Volumen) mittels Filtration gewaschen und das Filtrat verworfen. Das Amberlite$^R$-XAD-2-Harz wurde nun in Methanol suspendiert (1/5; Gewicht/Volumen), die Suspension filtriert und das Filtrat, welches das opiataktive Peptid enthielt, am Rotationsverdampfer eingedampft.

#### Schritt 4: Hochdruck-Flüssigkeitschromatographie (HPLC)

Ein Waters Chromatograph (Waters Millford, Massachusetts), ausgerüstet mit 2 Pumpen (6000 A), einem Gradienten Programmier-Gerät (660) und einem UV-Detektor/254 nm (440) wurde bei den folgenden Schritten benutzt. Alle Proben wurden vor der Injektion in das HPLC-System durch Mikrofilter (Millipore) filtriert. Die Injektionsvolumina betrugen 100—300 Microliter. Es wurden nur Lösungsmittel von p.a.-Qualität verwendet. Die Eluate wurden in Fraktionen von einem oder zwei Millilitern gesammelt. Nach dem Abdampfen der Fraktionen am Rotationsverdampfer oder nach ihrer Lyophilisierung wurden die jeweiligen Rückstände in Wasser aufgenommen und deren opiatartige Wirkung getestet.

Die Fraktionen mit opiatartig wirkendem Material aus einem HPLC-Reinigungsschritt wurden vereinigt, gefriergetrocknet und im nächstfolgenden Schritt weiter verarbeitet. Die bei der HPLC verwendeten Säulen wurden nach jedem Lauf mit 100% Methanol durchgespült. Um die Überladung der Säulen mit Probenmaterial zu vermeiden, wurde das Material eines bestimmten Reinigungsschrittes in bis zu zehn getrennten Einzelläufen chromatographiert und die Fraktionen der Einzelläufe, die Opiataktivität aufwiesen, wieder für den nächsten Schritt vereinigt.

#### HPLC/Stufe 1:

Das Material mit Opiataktivität aus Schritt 3 wurde in 0,1 N Essigsäure/Methanol (1/1; v/v) aufgenommen, auf eine $\mu$-Bondapak C 18-Säule (I.D. 7,9 mm; Länge 30 cm) aufgegeben und mit 0,1 N Essigsäure/Methanol eluiert. Die Elutionsgeschwindigkeit betrug 2,5 ml/Min. Die Opiataktivität wurde zwischen 6 und 11 Min. eluiert.

HPLC/Stufe 2:

Das Material mit Opiataktivität aus Stufe 1 wurde in Äthanol/Wasser (3/97; v/v) aufgenommen und wiederum auf eine $\mu$-Bondapak C 18-Säule aufgebracht. Als Eluens diente Äthanol/Wasser (3/97; v/v). Die Flußgeschwindigkeit betrug 3 ml/Min. Innerhalb einer Zeit von 30 Min. wurde keine Opiataktivität eluiert. Erst im folgenden linearen 20-minütigen Gradienten von Äthanol/Wasser (3/97; v/v) auf 100% Äthanol konnte die Opiataktivität in den Fraktionen festgestellt werden, die innerhalb der 12. und 13. Minute nach Start des Gradienten gesammelt wurden.

HPLC/Stufe 3:

Das opiataktive Material der Stufe 2 wurde gefriergetrocknet, in einer geringen Menge Chloroform/Methanol (1/1; v/v) aufgenommen und auf eine Mikroporasil-Säule (I.D. 7,9 mm; Länge 30 cm) aufgegeben. Als Eluens diente Chloroform/Methanol (1/1; v/v) bei einer Flußgeschwindigkeit von 2,5 ml/Min. Die Opiataktivität wurde zwischen 5,5 und 8,5 Min. eluiert.

HPLC/Stufe 4:

Das opiataktive Material aus Stufe 3 wurde in Wasser aufgenommen und auf die Mikroporasil-Säule gegeben. Während einer 10-minütigen Chromatographie unter Benutzung von Wasser als Eluens (Flußgeschwindigkeit 2,5 ml/Min.) wurde keine Opiataktivität eluiert. Danach wurde ein 10-minütiger linearer Gradient von 100% Wasser auf 100% Methanol gefahren. Opiataktives Material, welches über die gesamte Spanne des Gradienten eluiert wurde, wurde gepoolt, abrotiert und der wässrige Bestandteil gefriergetrocknet.

HPLC/Stufe 5:

Das im Gradienten eluierte opiatartig wirkende Material von Stufe 4 wurde in Wasser/Methanol (1/1; v/v) aufgenommen, auf die Mikroporasil-Säule aufgebracht und mit diesem Eluens bei einer Flußgeschwindigkeit von 2,5 ml/Min. eluiert. Die Elutionszeit der Opiataktivität betrug 5,7 bis 7,2 Min.

Schritt 5: Gelfiltration

Das opiatartig wirkende Material des letzten HPLC-Schrittes wurde auf eine BIO-GEL P 2-Säule (—400 mesh) (I.D. 1,0 cm; Länge 135 cm) aufgebracht und mit Trifluoressigsäure/Wasser (0,04%; v/v) eluiert. Die Flußgeschwindigkeit betrug 5,5 ml/Std. Die UV-Absorption des Eluates wurde bei 220 und 280 nm Wellenlänge gemessen und registriert. Das Elutionsvolumen des opiataktiven Materials lag zwischen 79 und 84 ml. Die UV-Absorption zeigte an dieser Stelle bei 220 und 280 nm einen scharfen Peak. Die spezifische Aktivität des eluierten Materials betrug 6500 Nanomole Normorphin-Aquivalente (pro g). Eine Nachreinigung konnte die spezifische Aktivität nicht mehr erhöhen.

Nachweis der opiatartigen Wirkung der teilweise oder vollständig gereinigten Verbindungen

Alle Überstände und Filtrate aus den Extraktions- und Adsorptionsprozenduren, sowie alle Fraktionen aus der Hochdruckflüssigkeitschromatographie und aus der Gel-Filtration wurden am Längsmuskel-Plexusmyentericus-Präparat des Meerschweinchenileums auf ihre opiatartige Wirksamkeit getestet. Die Proben wurden dazu vorher entweder abgedampft oder gefriergetrocknet. Nach der Aufnahme der Rückstände in Wasser wurden Teile dieser Proben dem Organbad zugesetzt und auf ihre hemmende Wirkung auf die elektrisch stimulierten Kontraktionen des Meerschweinchendarmpräparates getestet. Die hemmende Wirkung der zu testenden Stoffe wurde als opiatspezifisch betrachtet, wenn diese nach Zugabe des spezifischen Opiatantagonisten Naloxon aufgehoben wurden, und nach weiterem Zusatz von Naloxon keine Hemmung durch die Probe mehr hervorgerufen wurde. Präparation und elektrische Stimulation des Meerschweinchendarms (Frequenz 0,1 Hz, Pulse 60 V, 0,5 ms) wurden durchgeführt, wie von Kosterlitz et al. (Kosterlitz H.W., Lydon, R.J., Watt, A.J. Brit. J. Pharmacol. 39, 398—413 (1970)) und Schulz und Goldstein (Schulz, R., Goldstein, A.J. Pharmacol. exp. Ther. 183 404—410 (1972)) beschrieben.

Zur Kontrolle der Effektivität der verschiedenen Reinigungsschritte wurde die spezifische Opiataktivität bestimmt: Dazu wurde die opiatartige Wirkung der verschiedenen Proben pro Gewichtseinheit bestimmt und diese Wirkung auf die Wirkung des Opiates Normorphin bezogen, welches jeweils unter den gleichen Bedingungen wie die Probe am Meerschweinchendarm getestet worden war. Die daraus resultierenden quantitativen Angaben sind deshalb in Nanomolen (n mol.) Normorpholin-Äquivalenten gemacht.

Charakteristika der isolierten Peptide mit opiatartiger Wirkung

Das opiataktive Material aus dem letzten Reinigungsschritt (Gelfiltration) wurde mit 6 N HCL 24 Stunden unter Vacuum erhitzt, um das Peptid zu spalten. Eine Aminosäureanalyse mit einem handelsüblichen Aminosäureanalysator (Biotronik) des hydrolyisierten Materials ergab: Tyr: 0,9; Pro: 2,7; Phe: 1,0; Gly: 1,1; Ile: 0,8. Dieses Ergebnis entsprach den theoretischen Werten der Aminosäuresequenz der $\beta$-Caseinkette: Tyr: 1; Pro: 3; Phe: 1; Gly: 1; Ile: 1. Die Zahlen beziehen sich auf Reste Pro Mol Peptid. Ein N-terminaler Abbau mit Phenyl-Senföl nach Edmann (Edmann, P. und Henschen, A. (1975) in: Protein sequence determination, Needleman, S.B., e d., 2 nd edn., pp 232—279, Springer-Verlag,

Berlin) ergab die Sequenz: Tyr-Pro-Phe-Pro-Gly-Pro-Ile. Dieses Heptapeptid erhielt den Namen β-Casomorphin-7. Eine Incubation von 3 mg des β-Casomorphin-7 mit 0,2 mg Carboxypeptidase (Typ Y, Boehringer) bei 37°C in 6 molarem (M) Acetatpuffer bei pH 5,5 führte zu einer massiven Steigerung der Opiataktivität während der ersten drei Stunden. Durch Carboxypeptidase-Einwirkung war also durch einen Abbau des Peptids von der C-terminalen Seite her ein kürzeres aktivieres Bruchstück erzeugt worden. Eine Auftrennung auf der schon im Schritt 5 benutzten BIO-GEL P 2-Säule erbrachte 3-Hauptkomponenten: Reste des Ausgangsmaterials (β-Casomorphin-7) eluierten zwischen 79 und 84 ml; nach 95 bis 100 ml eluierte ein Material, dessen spezifische Opiataktivität um den Faktor 10 bis 20 höher lag als die des β-Casomorphin-7.

Eine Hydrolyse und ein nachfolgender Abbau nach Edmann (s.o.) ergab die Sequenz Tyr-Pro-Phe-Pro-Gly. Dieses neugeschaffene Pentapeptid erhielt den Namen β-Casomorphin-5.

Als Nachweis der Resistenz gegenüber Proteasen sei ein Beispiel aufgeführt: 0,3 mg β-Casomorphin-7 wurden in 0,6 ml Tris-Puffer pH 8 mit 0,02 mg Pronase E (Merck) bei 37°C 1 bis 4 Stunden incubiert. Nach Beendigung der Incubation wurde das Gemisch zwecks Zerstörung der Pronase 15 Min. bei 95°C erhitzt. Ein Vergleich mit einer Kontrollprobe ohne Zusatz des Enzyms erbrachte bei dem anschließenden Opiattest keinerlei Verluste an opiataktivität, während eine Probe mit Enzym und Methionin-Enkephalin die Opiataktivität völlig verlor.

Weitere Opiateigenschaften der erfindungsgemäßen Peptide

Die Opiateigenschaften der erfindungsgemäßen Peptide umfassen neben der Wirkung auf isoliert Organe und deren Präparationen (Meerschweinchendarm) auch Wirkungen auf das Zentralnervensystem. Dies geht aus einem Versuch hervor, in welchem solche Peptide in das Ventrikelsystem von Ratten injiziert wurden. 30 bis 45 Min. nach Injektion von 400 bis 1200 Mikrogramm (in 10 Mikroliter gelöst) eines erfindungsgemäßen Peptids (β-Casomorphin-7) in das Ventrikel-System zeigten die Ratten eine starke Analgesie (herabgesetzte Schmerzempfindlichkeit), welche nach subkutaner Injektion von 10 mg pro kg Körpergewicht des spezifischen Opiatantagonisten Naloxon aufgehoben wurde.

Beispiel II

Synthese eines erfindungsgemäßen Pentapeptids der Sequenz: Tyr-Pro-Phe-Pro-Gly-Methylester.

A Syntheseplan: Die Aminokomponente wird bei −15°C oder niedriger in Dimethylformamid (DMF) mit einem 0,5 molaren Überschuß an gemischtem Anhydrid einer Z-Aminosäureisobutylcarbonsäure (wobei Z als Schutzgruppe dient und einen N-Benzyloxycarbonyl-Rest darstellt) 2—4 Stunden umgesetzt.

Das gemischte Anhydrid wird in DMF bei −15°C oder niedriger in 10 bis 15 Minuten unter Einsatz eines 6%-igen Überschusses am Z-Aminosäurederivat und N-Methylmorpholin über den Chlorameisensäureisobutylester gebildet. Der Überschuß an gemischtem Anhydrid wird dann zerstört. Bei 0°C wird der pH des Reaktionsproduktes mit wässriger, gesättigter $KHCO_3$-Lösung auf 8 eingestellt und und 30 Minuten bei 0°C gerührt.

Die Peptide wurden mit Ethylacetat extrahiert. Das Ethylacetat/Peptidgemisch wurde, um das Z-Aminosäure-Kaliumsalz zu entfernen, 3 Mal mit Natriumchlorid/Wasser, 3 Mal mit Wasser gewaschen und abgedampft. Das so erhaltene Peptid, welches noch die Schutzgruppe Z trägt, wurde in Methanol hydriert. Dabei wurden 100—500 mg Pd/Aktivkohle Katalysator pro mmol Peptid zugesetzt. Die $CO_2$-Abspaltung wurde mit $Ba(OH)_2$-Lösung kontrolliert. Der Katalysator wurde abfiltriert (Schleicher & Schüll Papierfilter N° 595), mit Wasser ausgiebig gewaschen und das Filtrat auf einem Rotationsverdampfer (Büchi, Rotavapor RE) eingedampft. Das gewünschte deblockierte Peptid ist im Rückstand.

B Synthese des Pentapeptids Tyr-Pro-Phe-Pro-Gly-Methylester (β-Casomorphin-5-OMet)

Stufe 1:

a) Herstellung des gemischten Anhydrids (Z-Phe-Pro-gemischtes Anhydrid)

640 mg (1,6 mM; = 6%-iger Überschuß) des Dipeptids Z-L-Phe-L-Pro (wobei Z ein N-Benzyloxycarbonyl-Rest ist, welcher als Schutzgruppe fungiert) werden in 20 ml Dimethylformamid (DMF) mit 200 μl (1,5 mM) Chlorameisensäureisobutylester bei −15°C nach Zusatz von 170 μl (1,6 mM) N-Methylmorpholin 15 Minuten umgesetzt.

b) Vorbereitung der Aminokomponente

125,6 mg (1,0 mM) Glycinmethylesterhydrochlorid werden in 20 ml DMF unter Zusatz von 110 μl (1 mM) N-Methylmorpholin bei −15°C aufgelöst.

Stufe 2:

Umsetzung des gemischten Anhydrids der Stufe 1 a mit der Aminokomponente 1 b. Das Z-Phe-Pro-gemischte Anhydrid wird mit dem Glycinmethylester in 40 ml DMF bei −15°C 4 Stunden umgesetzt.

$$\text{Z-Phe-Pro-gem. Anhydrid} + \text{Gly-Metester} \xrightarrow[\text{4 h}]{-15°\text{C}} \text{Z-Phe-Pro-Gly-Metester}$$

Vor der Aufarbeitung wird der 50%-iger Überschuß an gemischtem Anhydrid zerstört. Bei 0°C wird der pH des Reaktionsproduktes mit wässriger, gesättigter $KHCO_3$-Lösung auf pH 8 eingestellt

und 30 Minuten bei 0°C gerührt. Danach wird das Peptid mit 50—100 ml Ethylacetat (EtAc) extrahiert; das EtAc/Peptidgemisch wird mit gesättigter, wässriger Natriumchlorid-Lösung gewaschen. Nach einem weiteren abschließenden Waschen mit Wasser wird die EtAc-Phase eingedampft.

Stufe 3:

Abspalten der Schutzgruppe durch Hydrierung.

Das Peptid wird in 30 ml Methanol gelöst und 100 mg Palladium auf Aktivkohle (Merck) zugegeben. Nach dem Verdrängen der Luft durch Stickstoff wird in das Reaktionsgefäß Wasserstoff eingeleitet. Die Hydrierung wird bei 25°C—30°C durchgeführt. Die Hydrierung ist beendet, wenn kein $CO_2$ mehr freigesetzt wird, d.h. wenn nach Kontrolle in wässriger Bariumhydroxid-Lösung kein Niederschlag mehr gebildet wird. Die Lösung wird filtriert, mit Wasser gewaschen und am Rotationsverdampfer einrotiert. Dieses verbleibende Zwischenprodukt wird dann in Stufe 4 als Aminokomponente eingesetzt.

Stufe 4:

a) Herstellung des gemischten Anhydrids (z-Progem. Anhydrid)

374 mg (1,5 mM) Z-L-Prolin werden in 15 ml DMF unter Zusatz von 170 $\mu$l (1,5 mM) N-Methylmorpholin gelöst und mit 180 $\mu$l (1,4 mM) Chlorameisensäureisobutylester bei —15°C 15 Minuten umgesetzt.

b) Umsetzung des gemischten Anhydrids aus der Stufe 4a mit der Animokomponente der Stufe 3.

$$\text{Z-L-Pro-Anydrid + Phe-Pro-Gly-Metester (in 15 ml DMF} \xrightarrow[4\ h]{-15°C} \text{Z-Pro-Phe-Pro-Gly-Metester.}$$

Die Zerstörung des überschüssigen gemischten Anhydrids, die Extraktionsschritte, die Hydrierung werden identisch die vorher beschrieben durchgeführt.

Das Endprodukt 4 b dient als Aminokomponente für die Stufe 5. Eine Kontrolle der Aminosäurezusammensetzung nach Hydrolyse ergab die richtige molare Aminosäurerelation dieses Peptids. Ein Test auf die Opiataktivität (Testmethode siehe Beispiele I) ergab keinerlei opiatartige Wirkung.

Stufe 5:

a) Bildung des gemischten Anhydrids (Z-Tyrgem. Anhydrid)

629,24 (1,4 mM) N,O-di-Z-L-Tyrosin werden in 15 ml DMF unter Zusatz von 165 $\mu$l (1,4 mM) N-Methylmorpholin gelöst und mit 175 $\mu$l (1,3 mM) Chlorameisensäureisobutylester bei —15°C 15 Minuten umgesetzt.

5 b) Umsetzung des gemischten Anhydrids aus Stufe 5 a mit dem Endprodukt der Stufe 4 b. Das Endprodukt der Stufe 4 b wird in 15 ml DMF gelöst und mit dem gemischten Anhydrid der Stufe 5 a bei —15°C 4 Stunden umgesetzt.

Die Zerstörung des überschüssigen gemischten Anhydrids, die Extraktion und die Hydrierung erfolgen wie oben beschrieben.

Das gewünschte Endprodukte Tyr-Pro-Phe-Pro-Gly-Methylester zeigt eine starke spezifische opiatartige Wirkung am Meerschweinchendarmpräparat. Eine Auftrennung der Reaktionsprodukt über Biogel P 2 (wie vorher beschrieben) ergab eine bestimmte Position der Opiataktivität.

Eine nach der sauren Hydrolyse durgeführte Aminosäureanalyse ergab ein richtiges molares Aminosäureverhältnis entsprechend dem $\beta$-Casomorphin-5. Es sei darauf hingewiesen, daß bereits die Aktivierung des opiatinaktiven Tetrapeptids (Stufe 4 b) durch Tyrosinkopplung in ein opiataktives Peptid den Nachweis der erfolgereichen Synthese darstellt.

**Patentansprüche**

1. Pharmakologisch aktive Peptide, insbesondere opiatartig wirkend, mit 3—8 Aminosäureresten der Formel

V-Pro-A-X

in der steht:

Pro — für Prolin
A — für jede beliebige Aminosäure, auch Prolin und Tyrosin
X — für

a) C-terminale Ester oder Amide des Tripeptides V-Pro-A
b) Pro-Y
Pro-B-Y
Pro-B-Pro-Y
Pro-B-Pro-C-Y
Pro-B-Pro-C-Pro-Y

wobei

B und C jede beliebige Aminosäure sein kann, auch Prolin und Tyrosin. Y steht für die freie Säurefunktion der jeweiligen C-terminalen Aminosäure oder deren Ester oder deren Amid.

V — für die N-terminale Aminosäure Tyrosin oder für das N-terminal endständige Tyrosin der allgemeinen Formel

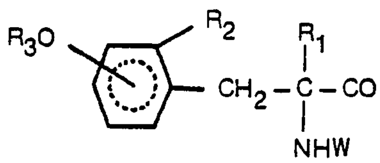

wobei im einzelnen bedeuten:

$R_1$ = Wasserstoff oder eine Alkylgruppe mit 1 bis 4 C-Atomen,

$R_2$ = Wasserstoff oder zusammen mit $R_1$ für eine Aethylenbrücke,

$R_3$ = Wasserstoff, eine Alkylgruppe mit 1 bis 4 C-Atomen oder eine $R_4CO$-Gruppe,

$R_4$ = einen gesättigten oder ungesättigten, geradkettigen oder verzweigten Alkylrest mit 1 bis 17-C-Atomen, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei die $R_3O$-Gruppe sich in meta- oder para-Stellung zum

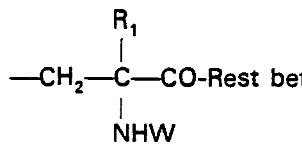 befindet,

W = Wasserstoff, Alkyl mit 1 bis 5 C-Atomen, Alkenyl mit 3 bis 5 C-Atomen, Cyclopropylmethyl, $R_4CO$—, H-Lys-, H-Phe- oder H-Tyr.

2. Pharmakologisch aktive Peptide, insbesondere opiatartig wirkend, nach Anspruch 1, dadurch gekennzeichnet, daß

A Phenylalanin

B Glycin

C Isoleucin

ist.

3. Pharmakologisch aktive Peptide, insbesondere opiatartig wirkend, nach einem der Ansprüche 1 und 2, dadurch gekennzeichnet, daß

a) das Phenylalanin der allgemeinen Formel

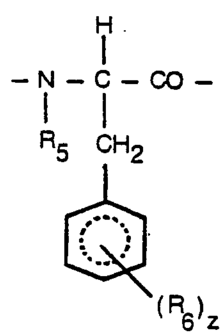

vorliegt, wobei im einzelnen bedeuten:

$R_5$ = Wasserstoff oder Alkyl mit 1 bis 4 C-Atomen,

$R_6$ = Wasserstoff, Fluor, Chlor, Brom, Nitro, Alkyl mit 1 bis 4 C-Atomen oder Alkoxy mit 1 bis 4 C-Atomen, z für 1 oder 2.

b) das Prolin der allgemeinen Formel

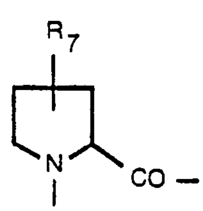

vorliegt, wobei im einzelnen bedeuten:

$R_7$ = Wasserstoff, eine Hydroxygruppe, eine Alkyl- oder Alkoxygruppe mit 1 bis 4 C-Atomen.
— am Stickstoff eine Alkyl- oder Alkoxygruppe mit 1 bis 4 C-Atomen gebunden ist.
— eine oder mehrere Ketogruppen in den Ring eingeführt sind.

4. Pharmakologisch aktive Peptide, insbesondere opiatartig wirkend, nach einem der Ansprüche 1—3, dadurch gekennzeichnet, daß die optisch aktiven Aminosäuren und/oder Aminosäurereste durch solche ersetzt sind, die stereochemisch die D-, L-, oder D/L-Konfiguration aufweisen.

5. Pharmakologisch aktive Peptide, insbesondere opiatartig wirkend, nach einem der Ansprüche 1—4, dadurch gekennzeichnet, daß jede(r) der optisch aktiven Aminosäuren und/oder Aminosäurereste vorzugsweise stereochemisch in der L-Konfiguration vorliegt.

6. Pharmakologisch aktive Peptide, insbesondere opiatartig wirkend, nach einem der Ansprüche 1—5, dadurch gekennzeichnet, daß diese aus natürlichen Quellen unter Anwendung allgemein üblicher Methoden erzeugt und anschließend isoliert werden.

7. Verfahren zur Herstellung von pharmakologisch aktiven Peptiden, insbesondere opiatartig wirkenden Peptiden der Formel

V-Pro-A-X

nach einem der Ansprüche 1—6, dadurch gekennzeichnet, daß diese aus in Tier- und Pflanzenreich vorkommenden Proteinen durch Enzyme freigesetzt und so aktiviert werden.

8. Pharmakologisch aktive Peptide, insbesondere opiatartig wirkend, nach einem der Ansprüche 1—7, dadurch gekennzeichnet, daß diese aus $\alpha$- und/oder $\beta$- und/oder $\varkappa$-Casein durch Enzyme freigesetzt werden und so in die aktive Form übergeführt werden.

9. Pharmakologisch aktive Peptide, insbesondere opiatartig wirkend, nach einem der Ansprüche 1—8, dadurch gekennzeichnet, daß die Enzyme pankreatischen Ursprungs sind.

10. Pharmakologisch aktive Peptide, insbesondere opiatartig wirkend, nach einem der Ansprüche 1—9, dadurch gekennzeichnet, daß die Enzyme Trypsin, Chymotrypsin, Pronase E, Thermolysin einzeln oder deren Gemische sind.

11. Pharmakologisch aktive Peptide, insbesondere opiatartig wirkend, nach einem der Ansprüche 1—10, dadurch gekennzeichnet, daß diese mittels eines oder mehrerer der folgenden Schritte aus einem enzymatisch verdauten Casein genommen werden:

a) einer Extraktion mit Chloroform/Methanol (65/35; Vol/Vol)

b) Adsorption des eingedampften Chloroform/Methanolextraktes aus wässriger Lösung an Aktivkohle. Desorption mit reinem Chloroform.

c) Adsorption des eingedampften Chloroform extraktes aus wässriger Lösung an Amberlite[R] (XAD—2, 300—1000 mesh). Desorption mittels Methanol.

d) weitere Reinigung des Methanolextraktes mittels einer Hochdruckflüssigkeitschromatographieanlage (HPLC) über eine sog. "reversed phase" Säule (Füllmaterial: $\mu$-Bondapak $C_{18}$, Waters) und über eine Silicagelsäule (Füllmaterial: $\mu$-Porasil, Waters) in lipophilen und/oder hydrophilen Lösungsmitteln.

e) Chromatographie auf einer Biogel P2-Säule.

12. Pharmakologisch aktive Peptide, insbesondere opiatartig wirkend, nach einem der Ansprüche 1—11, dadurch gekennzeichnet, daß durch Inkubation mit Carboxypeptidase (Y, Boehringer) die opiatartige Wirkung verstärkt wird.

13. Pharmakologisch aktive Peptide, insbesondere opiatartige wirkend, nach einem der Ansprüche 1—12, dadurch gekennzeichnet, daß diese mit einem in der Peptidchemie und/oder in der Natur üblichen Verfahren synthetisiert werden.

14. Pharmakologisch aktive Peptide, insbesondere opiatartig wirkend, nach einem der Ansprüche 1—13, dadurch gekennzeichnet, daß das Knüpfen der Peptidbindung zwischen den einzelnen Aminosäuren und/oder den kleineren Peptidfragmenten mit den üblichen Methoden erfolgt.

15. Arzneimittel und Tierarzneimittel, dadurch gekennzeichnet, daß diese die in Ansprüchen 1—14 charakterisierten pharmakologisch aktiven, insbesondere opiatartig wirkenden, Peptide und/oder deren Derivate und/oder deren Salze enthalten.

16. Pharmakologisch aktive Peptide, insbesondere opiatartig wirkend, nach einem der Ansprüche 1—14 zur Verwendung als Diagnostica.

17. Pharmakologisch aktive Peptide, insbesondere opiatartige wirkend, nach einem der Ansprüche 1—16 zur Verwendung als Antigene (nach Kopplung an Thyreoglobulin oder an andere makromoleculaire Eiweißkörper) zur Erzeugung von Antikörpern im Säugetierorganismus.

**Revendications**

1. Des peptides pharmacologiques actives, spécialement en agissant de manière d'opium, avec 3—8 résidus d'amino-acides de formule

V-Pro-A-X

dans laquelle est
Pro = proline
A = chaque quelconque amino-acide, aussi proline et tyrosine
X = a) des esters C-terminals ou des amides du tripeptide V-Pro-A
b) Pro-Y
Pro-B-Y
Pro-B-Pro-Y
Pro-B-Pro-C-Y
Pro-B-Pro-C-Pro-Y
où

B et C peut être chaque quelconque aminoacide aussi proline et tyrosine. Y représente la fonction d'acide libre d'aminoacide choisi C-terminal ou leur ester ou leur amide.

V = l'amino-acide N-terminal tyrosine
= tyrosine N-terminal, positionné au bout, de formule générale

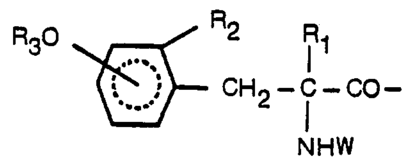

Dans cette formule générale est indiqué en détail:
$R_1$ est un atome d'hydrogène ou un groupe alkyle en $C_1$ à $C_4$,
$R_2$ est un atome d'hydrogène ou en ensemble avec $R_1$ und liaison d'éthylene,
$R_3$ est un atome d'hydrogène, un groupe alkyle en $C_1$ à $C_4$ ou un groupe de $R_4CO$—,
$R_4$ est un résidu d'alkyle, qui est saturé ou non saturé, en chaîne droite ou bifurqué en $C_1$ à $C_{17}$, un résidu de phényle ou de phénylalkyle en $C_7$ à $C_{12}$ dans lequel les résidus de phényle peuvent être substitués par 1 ou 2 substituents de la série d'halogènes, alkyle en $C_1$ à $C_4$ ou alkoxy en $C_1$ à $C_4$, où le groupe de $R_3O$ est en meta- ou para-position à

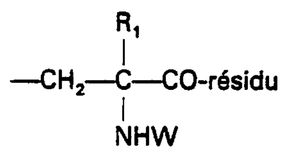

Dans cette formule peut être
W — un atome d'hydrogène, alkyle en $C_1$ à $C_5$, alcényle en $C_3$ à $C_5$, cyclopropylméthyle, $R_4CO$—.

2. Des peptides pharmacologiques actives, spécialement en agissant de manière d'opium, suivant revendication 1, caractérisés en ce que
A est phenylalanine
B est glycine
C est isoleucine

3. Des peptides pharmacologiques actives, spécialement en agissant de manière d'opium, suivant l'une des revendications 1 à 2, caractérisés en ce que
a) le phénylalanine de formule générale

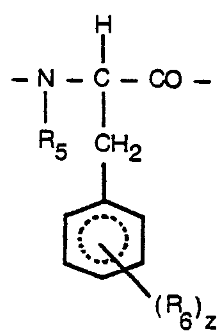

est présent, dans laquelle l'intention individuelle est:
$R_5$ est un atome d'hydrogène ou alkyle en $C_1$ à $C_4$,
$R_8$ est un atome d'hydrogène, fluorine, chlorine, bromine, nitro, alkyle en $C_1$ à $C_4$ ou alkoxy en $C_1$ à $C_4$, z est 1 ou 2.

b) le proline de formule générale

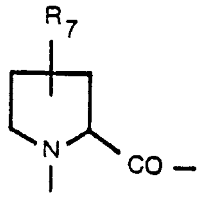

est présent, dans laquelle l'intention individuelle est:

$R_7$ représente un atome d'hydrogène, un groupe d'hydroxy, un groupe d'alkyle ou un groupe d'alkoxy en $C_1$ à $C_4$,

— à nitrogène est attaché un groupe d'alkyle ou un groupe d'alkoxy en $C_1$ à $C_4$,

— un groupe ou plusieurs groupes de keto sont introduits dans le rond.

4. Des peptides pharmacologiques actives, spécialement en agissant de manière d'opium, suivant l'une des revendications 1 à 3, caractérisés en ce que chaque des amino-acides optiques actives et/ou des résidus d'amino-acides a la configuration chimique D-, L-, ou D/L, excepté le tyrosine N-terminal, quel est présent seulement à L-forme.

5. Des peptides pharmacologiques actives, spécialement en agissant de manière d'opium, suivant l'une des revendications 1 à 4, caractérisés en ce que chaque des amino-acides optiques actives et/ou des résidus d'amino-acides est présent préférablement dans la configuration chimique L.

6. Des peptides pharmacologiques actives, spécialement en agissant de manière d'opium, suivant l'une des revendications 1 à 5, caractérisés en ce que ceux-ci sont puisés aux sources naturelles sous utilisation des méthodes générales usuels en sont puis isolés.

7. Procédé de préparation des peptides pharmacologiques actives, spécialement en agissant de manière d'opium, de formule

V-Pro-A-X

suivant l'une des revendications 1 à 6, caractérisés en ce que ceux-ci sont dégagés par des enzymes de proteins, quels sont présents dans le règne animal et plantes et sont activés de cette manière.

8. Des peptides pharmacologiques actives, spécialement en agissant de manière d'opium, suivant l'une des revendications 1 à 7, caractérisés en ce que ceux-ci sont dégagés par des enzymes de $\alpha$- et/ou $\beta$- et/ou $\varkappa$-casein et sont transférés à forme active.

9. Des peptides pharmacologiques actives, spécialement en agissant de manière d'opium, suivant l'une des revendications 1 à 8, caractérisés en ce que les enzymes sont d'origine de pankreas.

10. Des peptides pharmacologiques actives, spécialement en agissant de manière d'opium, suivant l'une des revendications 1 à 9, caractérisés en ce que les enzymes trypsine, chymotrypsine, pronase E, thermolysine sont en particulier ou leurs mélanges.

11. Des peptides pharmacologiques actives, spécialement en agissant de manière d'opium, suivant l'une des revendications 1 à 10, caractérisés en ce que ceux-ci moyennant l'une ou plusieurs des pas suivants sont pris d'un casein digéré enzymatique

a) d'une extraction de chloroforme/méthanole (65/35; vol/vol)

b) adsorption d'extracte de chloroforme/méthanole evaporé de la solution aqueuse à carbon actif. Désorption avec chloroforme pure.

c) adsorption d'extracte evaporé de chloroforme d'une solution aqueuse à Amberlite[R] (XAD—2, 300—1000 mesh), Désorption moyennant méthanole.

d) rectification suivante d'extracte de méthanole moyennant d'une installation de chromatographie de liquide avec haute pression (HPLC) sur une colonne qu'on dit "reversed phase" (materiaux de remplissage: $\mu$-Bondapak $C_{18}$, Waters) et sur une colonne de silicagel (materiaux de remplissage: $\mu$-Porasil, Waters) dans solvants lipophiles et/ou hydrophiles.

e) Chromatographie sur une colonne de Biogel P2.

12. Des peptides pharmacologiques actives, spécialement en agissant de manière d'opium, suivant l'une des revendications 1 à 11, caractérisés en ce que l'effet agissant de manière d'opium est fortifié par incubation avec carboxypeptidase (Y, Boehringer).

13. Des peptides pharmacologiques actives, spécialement en agissant de manière d'opium, suivant l'une des revendications 1 à 12, caractérisés en ce que ceux-ci sont préparés par un procédé de préparation classique de la chimie des peptides et/ou par un procédé, qu'il est usuel dans la nature.

14. Des peptides pharmacologiques actives, spécialement en agissant de manière d'opium, suivant l'une des revendications 1 à 13, caractérisés en ce que le couplage de la liaison peptidique entre les amino-acides particuliers et/ou entre plus petits fragments peptidiques résulte des méthodes classiques.

15. Des médicaments humains et des médicaments vétérinaires, caractérisés en ce que ceux-ci contient les peptides pharmacologiques actives, spécialement en agissant de manière d'opium, comme caractérisés en revendications 1 à 14, et/ou leurs dérivés et/ou leurs sels.

16. Des peptides pharmacologiques actives, spécialement en agissant de manière d'opium, suivant l'une des revendications 1 à 14 pour application diagnostique.

17. Des peptides pharmacologiques actives, spécialement en agissant de manière d'opium, suivant l'une des revendications 1 à 16, pour application qu'antigènes (après couplage à thyreoglobuline ou à autres corps d'albumine) à procréation des corps d'anti dans l'organisme de mammifère.

**Claims**

1. Pharmacologically active peptides, especially with opiate effect, with 3—8 amino acid residues of the formula

V-Pro-A-X

in which
Pro — represents proline
A — represents any amino acid, including proline and tyrosine
X — represents
a) esters or amides of the C-terminal amino acid of the V-Pro-A tripeptide
b) Pro-Y
Pro-B-Y
Pro-B-Pro-Y
Pro-B-Pro-C-Y
Pro-B-Pro-C-Pro-Y

wherein
B and C represent any amino acid including proline and tyrosine and Y represents the free acid function of the respective C-terminal amino acid or an ester or amide thereof.

V — represents an N-terminal amino acid tyrosine or an N-terminal end-positioned tyrosine of the general formula

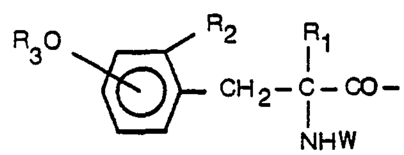

whereby the individual meaning is:
$R_1$ represents hydrogen or an alkyl group with 1 to 4 C-atoms,
$R_2$ represents hydrogen or together with $R_1$ represents an ethylene bond,
$R_3$ represents Hydrogen, alkyl group with 1 to 4 C-atoms or a $R_4CO$-group,
$R_4$ represents a saturated or unsaturated straight-chain or branched alkyl residue with 1 to 17 C-atoms, a phenyl residue or a phenylalkyl residue with 7 to 12 C-atoms, in which the phenyl residues can be substituted by 1 or 2 substituents of the halogen series, alkyl with 1 to 4 C-atoms or an alkoxy with 1 to 4 C-atoms, in which the $R_3O$-group is in meta or para position to

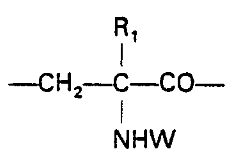 remainder

W represents hydrogen, alkyl with 1 to 5 C-atoms, alkenyl with 3 to 5 C-atoms, cyclopropylmethyl, $R_4CO$—.

2. Pharmacologically active peptides, especially with opiate effect, according to claim 1, characterized that
A is phenylalanine
B is glycine
C is isoleucine

3. Pharmacologically active peptides, especially with opiate effect, according to one of the claims 1 and 2, characterized in that the peptide contains

a) a phenylalanine of the general formula

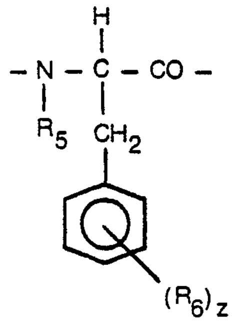

in which the individual meaning is:

$R_5$ represents hydrogen or alkyl with 1 to 4 C-atoms,

$R_6$ represents hydrogen, fluorine, chlorine, bromine, nitro, alkyl with 1 to 4 C-atoms or alkoxy with 1 to 4 C-atoms, z is 1 or 2.

b) a proline of the general formula

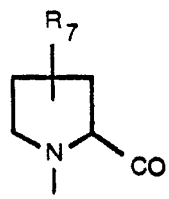

in which the individual meaning is:

$R_7$ represents hydrogen, a hydroxy group, an alkyl group or alkoxy group with 1 to 4 C-atoms,

— at the nitrogen is bonded an alkyl group or alkoxy group with 1 to 4 C-atoms,

— one or more keto groups is positioned on the ring.

4. Pharmacologically active peptides, especially with opiate effect, according to one of the claims 1—3, characterized in that each of the optically active amino acids and/or amino acid residues has the D-, L-, or D/L-stereochemical configuration; except the N-terminal tyrosine, which is only present in the L-configuration.

5. Pharmacologically active peptides, especially with opiate effect, according to one of the claims 1—4, characterized in that each of the optically active amino acids and/or amino acid residues preferably is present stereochemically in the L-configuration.

6. Pharmacologically active peptides, especially with opiate effect, according to one of the claims 1—5, characterized in that they are created from natural sources by using customary methods and then are isolated.

7. Process to synthesize pharmacologically active peptides, especially peptides with opiate effect of the formula

V-Pro-A-X

according to one of the claims 1—6, characterized in that these are fixed by enzymes from proteins existing in the animal and vegetable world and thus are activated.

8. Pharmacologically active peptides, especially with opiate effect, according to one of the claims 1—7, characterised in that these are released, by enzymes, from the $\alpha$- and/or $\beta$- and/or $\chi$-casein and thus are converted into the active stage.

9. Pharmacologically active peptides, especially with opiate effect, according to one of the claims 1—8, characterized in that the enzymes are of pancreatic origin.

10. Pharmacologically active peptides, especially with opiate effect, according to one of the claims 1—9, characterized in that the enzymes trypsine, chymotrypsine, Pronase E, thermolysine are used individually or in mixtures.

11. Pharmacologically active peptides, especially with opiate effect, according to one of the claims 1—10, characterized in that these by means of one or several of the following steps are released from a casein which was digested enzymatically:

a) extraction with chloroform/methanol (65/35; vol/vol)

b) adsorption of the evaporated chloroform/methanol extract from aqueous solution of activated carbon. Desorption with pure chloroform.

c) Adsorption of the evaporated chloroform extract from an aqueous solution on Amberlite[R] (XAD—2, 300—1000 mesh). Desorption by means of methanol.

d) further purification of the methanol extract by means of a high pressure liquid chromatography

apparatus (HPLC) via a so-called "reversed phase" column (filling material: μ-Bondapak $C_{18}$, Waters) and via a silica gel column (filling material: μ-Porasil, Waters) in lipophilic and/or hydrophilic solvents.

e) Chromatography on a Biogel P 2 column.

12. Pharmacologically active peptides, especially with opiate effect, according to one of the claims 1—11, characterized in that the opiate-like activity is increased by incubation with carboxypeptidase (Y, Boehringer).

13. Pharmacologically active peptides, especially with opiate effect, according to one of the claims 1—12, characterized in that these are synthesized by a method customary in peptide chemistry and/or in nature.

14. Pharmacologically active peptides, especially with opiate effect, according to one of the claims 1—13, characterized in that formation of the peptide bond between the individual amino acids and/or the smaller peptide fragments is accomplished with the customary methods.

15. Human and animal medication, characterized in that these contain the pharmacologically peptides, especially with opiate effect, characterized as in the claims 1 to 14 and/or their derivatives and/or their salts.

16. Pharmacologically active peptides, especially with opiate effect, according to one of the claims 1—14, characterized in that these are used as diagnostics.

17. Pharmacologically active peptides, especially with opiate effect, according to one of the claims 1—16, characterized in that the peptides, after bonding to thyreoglobuline or other macromolecular albumen bodies are used as antigens for the formation of antibodies in a mammal organism against the peptides described in the claims 1 to 16.